# EUROPEAN PATENT APPLICATION

(11) **EP 2 166 002 A1**
(43) Date of publication of application: **24.03.2010**
(21) Application number: 08016622.6
(22) Date of filing: 22.09.2008
(51) Int. Cl.: C07D 213/81, C07C 209/52

(54) **Chiral organic catalysts for the enantioselective reduction of n-alkyl and n-benzyl-substituted alicyclic and cyclic imines**

(71) Applicant: UNIVERSITA' DEGLI STUDI DI MILANO, 20122 Milano (IT)
(72) Inventor: Benaglia, Maurizio, 22071 Cadorago (CO) (IT); Guizzetti, Stefania, 20143 Milano (IT)
(74) Representative: Minoja, Fabrizio

(57) **Abstract**

Chiral organic catalysts of formula (I) 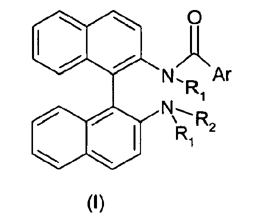 wherein ar, R₁ and R₂ are as defined in the description are herein disclosed. these catalysts promote the enantioselective reduction of *n*-alkyl and *n*-benzyl- substituted alicyclic and cyclic imines in the presence of trichlorosilane, avoiding the use of metal chitalysts or of metal-free catalysts which suffer from stability problems. the invention also relates to the preparation of the catalysts and to a process for the reduction on *n*-alkyl and *n*-benzyl substituted alicyclic and cyclic imines in which the imines are prepared in situ.

## Description

### Field of the invention

The present invention relates to chiral organic catalysts for the enantioselective reduction of *N*-alkyl and *N*-benzyl- substituted alicyclic and cyclic imines, a process for the preparation of the catalysts and a process for the reduction of *N*-alkyl substituted alicyclic and cyclic imines to chiral amines.

### Background of the invention

The reduction of carbon-nitrogen double bonds is of remarkable interest in the preparation of pharmaceutical products, agrochemicals, fragrances and precursors of biologically active molecules.

Catalytic systems currently available for the enantioselective reduction of carbon-nitrogen double bonds are either organometallic compounds (Catalytic Asymmetric Synthesis, 2nd ed.: Ojima, I., Ed.: Wiley: New York, 2000 and H.U. Blaser Adv. Synth. Catal., 2002, 344, 17) or metal-free compounds.

The former are quite expensive, due to the fact that they usually consist of an enantiomerically pure ligand (whose synthesis may be costly and troublesome) and by a metal, which is often an expensive one. Organometallic catalysts may cause product metal contamination, which represents a problem especially in the case of pharmaceutical compounds; metals may also require particular disposal procedures. In particular, very few catalysts efficiently promote enantioselective reduction of *N*-benzyl and *N*-alkyl ketimines. Highly expensive ruthenium-based or iridium-based organometallic catalysts have been reported to enantioselectively reduce *N-*alkyl substituted imines by hydrogenation, which often requires high pressure and sometimes even high temperature; for this reason, carrying out such a process on an industrial scale would be troublesome.

Examples of organic catalysts are chiral binaphthol-derived phosphoric acids (Review: T. Akiyama, J. Itoh, K. Fuchibe Adv. Synth. Catal.. 2006, 348, 999), which have successfully been employed in the reduction of imines and in reductive amination processes. These metal-free catalysts often have high molecular weight (> 600 Da) and require the Hantzsch ester as reducing agent, a dihydropyridine compound which must be separated from the reaction product.

None of the above catalysts has been reported to enantioselectively reduce alicyclic *N*-benzyl or *N*-alkyl substituted imines.

Metal-free catalysts have also been developed to steroselectively reduce imines with trichlorosilane. In this case the reducing agent, trichlorosilane, is activated by coordination with a Lewis base, such as *N*,*N*-dimethylformamide, acetonitrile or a trialkylamine, to generate a hexacoordinated hydridosilicate, the actual active reducing agent which operates under mild conditions. For example, some *N*-formyl derivatives of amino acids are able to promote the reduction with high enantioselectivity (Malkov, A. V. et al. Tetrahedron 2006, 62, 264), but they suffer from stability problems and some of them are expensive. Recently, other chiral Lewis bases in the form of *N*-formamides were prepared from (S)-pipecolinic acid and enantiopure 2-amino-1,2-diphenylethanol (both commercially available) and successfully employed in the reduction of ketimines (Wang, Z. Org. Lett. 2006, 25, 3045). Also quinoline-, isoquinoline- and pyridine-derived chiral oxazolines can efficiently promote the addition of trichlorosilane to ketones and imines (Malkov, A.V. et al Angew. Chem. Int. Ed. 2006, 45, 1432). It has also been recently disclosed that stereoselective reduction with trichlorosilane of a broad range of *N*-aryl ketimines can be catalyzed by a chiral sulfinamide which contains a stereogenic sulfur atom (Pei, D. Org. Lett. 2006, 25, 5913). However, many of the catalysts mentioned hereinbefore are poorly stable, difficult to prepare and their chemical and stereochemical efficiency is variable. Finally, it has recently been disclosed that *N-*picolinoylpyrrolidine derivatives activate trichlorosilane in the reduction of aromatic imines (Onomura, O. et al. Tetrahedron Lett. 2006, 47, 3751); however, the reduction of *N*-alkyl substituted ketimines occurred only two cases, where the catalyst proved stereochemically inefficient, the product being obtained as racemic mixture [Malkov, A. V. et al. Tetrahedron 2006, 62, 264] or with low enantioselectivity (Wang, Z. Org. Lett. 2006, 25, 3045).

There is therefore the need to provide efficient catalysts for the enantioselective reduction of ketimines, in particular of *N*-alkyl ketimines.

### Description of the invention

In a first embodiment, the present invention provides compounds of general formula (I) 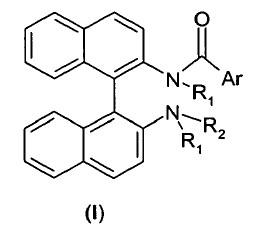 wherein:
R₁ is alkyl or optionally substituted benzyl;
R₂ is alkyl or COR₃, in which R₃ is optionally substituted alkyl or aryl, such as phenyl, or pyridine, quinoline or isoquinoline optionally substituted with one or more halogens, alkyl or alkoxy groups;
Ar is a pyridine, quinoline or isoquinoline residue optionally substituted with one or more halogens, alkyl or alkoxy groups which may be the same or different from one another.

Usually, in the compounds of formula (I), a pyridine residue is pyridin-2-yl, a quinoline residue is quinolin-2-yl and an isoquinoline residue is isoquinolin-1-yl.
In the present description:
"alkyl" means straight or branched (C₁-C₄)alkyl;
"optionally substituted benzyl" means benzyl optionally substituted with one or more alkyl or alkoxy, or with one or more halogens, which can be the same or different from one another;
"alkoxy" means straight or branched (C₁-C₄)alkoxy;
"halogen" means a halogen atom selected from fluorine, chlorine, bromine and iodine;
"optionally substituted alkyl" means straight or branched (C₁-C₄)alkyl optionally substituted with one or more halogens, which can be the same or different from one another.

In a second aspect, the invention provides a process for preparing the compounds of formula (I). The compounds of formula (I) wherein R₁ is alkyl or optionally substituted benzyl and R₂ is alkyl can be prepared by reaction of a chiral diamine of formula (IIa): 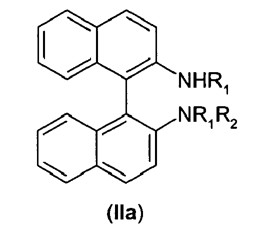 with an equimolar amount of an activated carboxylic acid Ar-COX, wherein Ar is as defined above and X is a carboxy-activating group.

The compounds of formula (I) wherein R₁ is alkyl or optionally substituted benzyl and R₂ is COR₃ can be prepared by reaction of a chiral diamine of formula (IIb) 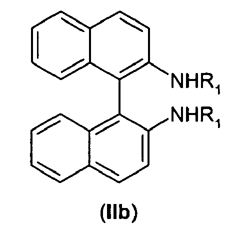 with an equimolar amount of an activated carboxylic acid Ar-COX followed by an equimolar amount of an activated carboxylic acid R₃COX, wherein R₃ is as defined above and X is a carboxy-activating group. When both Ar and R₃ are pyridine, the reaction can be carried out in one single step, using two molar amounts of activated picolinic acid.

Carboxy-activating groups are well known to skilled chemists; however, suitable carboxy-activating groups are for example halogens or sulfonyl groups, such as chlorine and methanesulphonyl; the activated carboxylic acid can be prepared *in situ* according to known procedures. The reaction can be carried out in an anhydrous organic solvent, such as chloroform, dichloromethane, tetrahydrofuran, dioxane, toluene, acetonitrile or DMF at a temperature ranging from -20°C to 100°C.

In a third aspect, the invention provides a process for reducing chiral *N*-alkyl and *N*-benzyl ketimines to the corresponding chiral amines which comprises the reduction of a chiral *N*-alkyl and *N*-benzyl ketimine with trichlorosilane in the presence of a catalytic amount of at least one compound of formula (I). In greater detail, the process of the invention allows to prepare chiral alicyclic amines of formula (III) 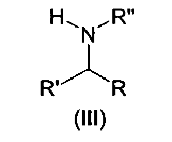 from *N*-alkyl or *N*-benzyl ketimines of formula (IV) 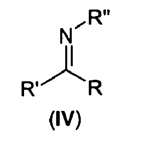 and cyclic chiral amines of formula (V) 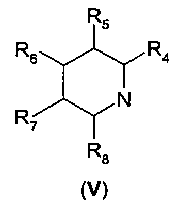 from cyclic imines of formula (VI)

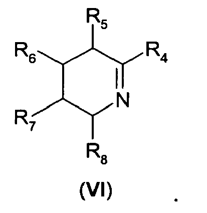

In formulae (III) and (IV), R, R' and R" have the following meanings:
R is straight or branched (C₁-C₄)alkyl;
R' is phenyl or naphthyl optionally substituted with one or more halogens, alkyl or alkoxy groups as defined above, which may be the same or different from one another; or R' is straight or branched (C₁-C₄)alkyl wherein the carbon chain is optionally interrupted by an oxygen atom;
R" is optionally substituted alkyl or benzyl as defined above.

In formulae (V) and (VI), R₄-R₈ are independently selected from hydrogen, straight or branched (C₁-C₄)alkyl or optionally substituted phenyl, or any two adjacent R₄-R₈ groups can be reciprocally linked to form a six-membered aromatic ring, in particular a benzyl ring and the remaining groups are as defined hereinbefore.

The reduction of a ketimine of formula (IV) or (VI) with trichlorosilane is carried out according to methods known to skilled chemists, usually using 0.1-0.01 mol/eq of compound of formula (I) and 3.5 mol/eq of trichlorosilane in a suitable organic solvent. Suitable organic solvents are, for example acetonitrile, toluene, tetrahydrofuran, dioxane, and in particular chlorinated solvents, namely dichloromethane and trichloromethane. The reaction is carried out in the cold, usually at temperatures ranging from - 45°C to +5°C. Although usually one compound of formula (I) is used, mixtures of two or more of them can be used to optimise the reaction.

In a particular embodiment of the invention, ketimines (IV) and (VI) are prepared *in situ* from the corresponding ketones; ketimines (IV) are prepared by reaction of a ketone RCOR' and an amine R"NH₂ wherein R, R' and R" are as defined hereinbefore, while ketimines (VI) are prepared by cyclization of a γ-amino-ketone of formula (VII): 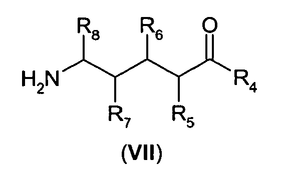 in which R₄-R₈ are as defined herein before.

The reduction of *N*-alkyl and *N*-benzyl ketimines, in particular of ketimines (IV) and (VI), in the presence of trichlorosilane can also be advantageously carried out with the (1*R*,2*S*)- or (1*S*,2*R*)-isomer of a compounds of formula (IX): 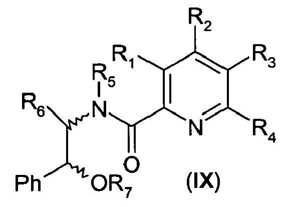 wherein
R₁ is H, halogen, preferably CI, Br, I or (C₁-C₄)straight or branched alkyl, preferably Me;
R₂ is H or halogen, preferably CI or Br;
R₃ is H or halogen, preferably CI;
R₄ is H or halogen, preferably CI;
R₅ is (C₁-C₄)straight or branched alkyl, preferably Me or *n*-Pr, or benzyl, optionally substituted with one or more halogens or (C₁-C₄)straight or branched alkyl groups, which can be the same or different from one another;
R₆ is (C₁-C₄)straight or branched alkyl, preferably Me or *i*-Pr, or R₆ can be linked to the adjacent 1-phenyl group to form a ring of formula A: 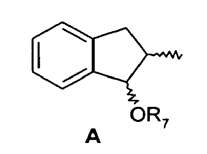
and R₇ is H, (C₁-C₄)straight or branched alkyl, preferably Me, or a CO-aryl group, wherein aryl is selected from phenyl, naphthyl, thiophenyl, furyl, oxazolyl, thiazolyl and pyridyl and is preferably 2-pyridyl.

The compounds of formula (IX), their synthesis and their use in the enantioselective reduction of *N*-aryl ketimines are disclosed in European patent application n. 07023240.0 filed on November 30, 2007. The compounds of formula (IX) in which R₁-R₄ are hydrogen, R₅ and R₆ are methyl and R₇ is hydrogen (*N*-methyl, *N*'-picolinoylephedrine) or methoxy and their use in the enantioselective reduction of *N*-aryl ketimines with trichlorosilane are disclosed by Zheng, H. et al. in Tetrahedron Lett., 48 (2007) 7934-7937.

Therefore, a further aspect of the present invention is the use of compounds (IX), alone or in admixture with one another, as catalysts in a process for the enantioselective reduction of *N*-alkyl and *N*-benzyl ketimines, in particular imines (IV) and (VI), in the presence of trichlorosilane. Also in this case the process can be advantageously carried out forming the ketimine *in situ*.

The present invention is advantageous in many aspects: the catalyst is metal-free and can be easily prepared through a one- or two-steps synthesis starting from commercially available cheap precursors and is highly stable, so it can be recovered after use; the reduction of imines (IV) and (VI) with trichlorosilane as reducing agent and the compounds of formula (I) as catalyst does not involve high costs, because trichlorosilane itself is cheap; hydrogenation or any other complex methodologies are not necessary and the catalyst operates at room pressure; finally, a simple aqueous work-up of the reaction mixture affords the desired products in high yields and high stereoselectivity.

The following experimental section illustrates the invention in greater detail.

### EXPERIMENTAL SECTION

### PART A - SYNTHESIS OF COMPOUNDS (I)

### Example 1. N,N'-dimethyl-N-(2-picolinoyl)-N'-benzoyl-1,1'-binaphthyl-2,2'-diamine

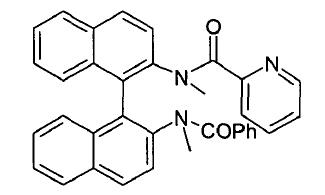

### Step a. Preparation of N,N'-dimethyl-N-(2-picolinoyl)-1,1'-binaphthyl-2,2'-diamine

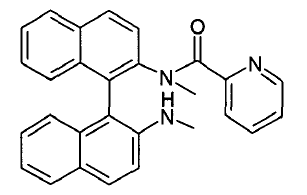

A solution of picolinic acid (2.2 mol equiv) in thionyl chloride (1 ml/mmol substrate) was refluxed under stirring for 2 hours, then the solvent was evaporated under vacuum; the residue was dissolved in THF (2 mL) with a few drops of DMF and was added to a solution of the chiral diamine *N,N'*-dimethyl-1,1'-binaphthyl-2,2'-diamine (IIa) of formula:

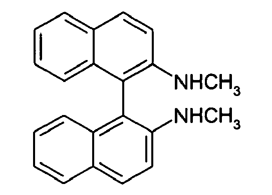

(1 mol equiv) and TEA (3 mol equiv) in THF; the reaction mixture was stirred for 12 hours under reflux. The organic phase was washed with an aqueous saturated solution of NaHCO₃ and brine, dried over Na₂SO₄, filtered and concentrated under vacuum to give a crude intermediate which was purified through flash chromatography.
Yield = 57%
MW = 417,50
¹H-NMR (300 MHz, CDCl₃): δ 2.6 (s, 3 H); 2.85 (s, 3 H); 6.51 (bs, 1 H), 6.70 (d, 2 H); 7.00-7.50 (m, 7 H), 7.80-8.00 (m, 7 H).
MS-ESI⁺: *m*/*z* 418 [M+H]⁺, *m*/*z* = 440 [M+Na]⁺.
IR (DCM): ν_{C=O} = 1685.01 cm⁻¹

### Step b. Preparation of the catalyst

Benzoyl chloride (2 mol equiv), dissolved in THF (2 mL) was added to a solution of the product of example 1 (1 mol equiv) and TEA (4 mol equiv) in THF and the reaction mixture was stirred for 12 hours under reflux. The organic phase was washed with an aqueous saturated solution of NaHCO₃ and brine, then dried over Na₂SO₄, filtered and concentrated under *vacuum* to give a crude product, which was purified through flash chromatography.
Yield = 77%
MW = 521,61
¹H-NMR (300 MHz, COCl₃): δ 2.6 (s, 3H), 2.9-3.0 (s, 3 H); 6.90 (m, 1 H); 7.00-7.30 (m, 12 H), 7.30-8.00 (m, 8 H).
MS-ESI⁺: *m*/*z* 522 [M+H]⁺, *m*/*z* = 544 [M+Na]⁺.
IR (DCM): ν_{C=O} = 1687.0, 1663.1 cm⁻¹

### Example 2. N,N'-dimethyl-N,N'-bis-(2-picolinoyl)-1,1'-binaphthyl-2,2'-diamine

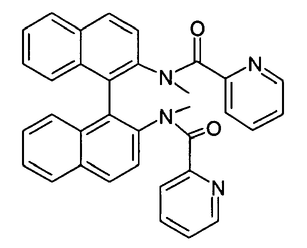

The procedure of example 1, step a), was followed, using 4.4 mol/eq picolinic acid.

### Example 3. N,N'-dimethyl-N,N'-bis-(6-methyl-2-picolinoyl)-1,1'-binaphthyl-2,2'- diamine

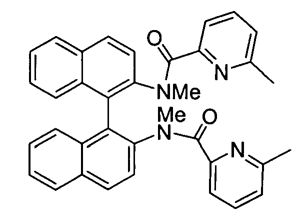

The procedure of example 1, step a), was followed, using 4.4 mol/eq of 6-methyl-2-picolinic acid.

### Example 4. N,N'-dimethyl-N,N'-bis-(3-methyl-2-picolinoyl)-1,1'-binaphthyl-2,2'- diamine

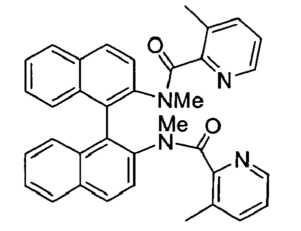

The procedure of example 1, step a), was followed, using 4.4 mol/eq of 3-methyl-2-picolinic acid.

### Example 5. N,N'-dimethyl-N'-methyl-N'-(-2-picolinoyl)-1'1-binaphthyl-2,2'- diamine

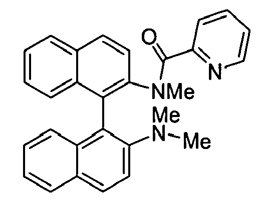

The procedure of example 1, step a), was followed, using 2.2 mol/eq of 2-picolinic acid which was reacted with *N,N*-dimethyl-1,1'-binaphthyl-2,2'-diamine (1 mol/eq).

### PART B - REDUCTION OF IMINES WITH COMPOUNDS (I)

### GENERAL PROCEDURE

A catalytic amount of compound of formula (I) (0.1-0.01 mol/eq) and an imine of formula (IV) or (VI) (1 mol/eq) are dissolved in the selected solvent and stirred at a suitable reaction temperature. Trichlorosilane (typically 3.5 mol/eq) is added and the mixture is stirred until completion of the reaction. A saturated solution of NaHCO₃ is then added, the organic phase is separated, dried over sodium sulfate and evaporated under reduced pressure. If necessary, the product is purified by flash chromatography.

### Example 6. Preparation of N-(1-phenylethyl)-butylamine

*N*-butylimine of acetophenone was reduced with trichlorosilane (1.5 - 3 mol/eq) in the presence of a catalytic amount (1%) of the compound of examples 1 and 2 with the solvents and under the conditions reported in table 1.

The compound of example 2 promoted the reduction in dichloromethane at 0°C in quantitative yield and 83% ee. Lowering the reaction temperature to -20°C enantioselectivity decreased to 66% without decrease of the chemical yield. Working in chloroform at °C *N-*(1-phenylethyl)-butylamine was isolated in 98% yield and 73% ee; decrease of the reaction temperature to -20°C lead to marginal loss of stereochemical efficiency (98% yield, 71 % ee).

The compound of example 1 promoted the reduction at 0°C in quantitative yield and 81 % ee in dichloromethane and 71 % ee in chloroform.

Noteworthy, catalyst loading can be lowered to 1% with only marginal loss (or no loss at all) of chemical efficiency and minor decrease in enantioselectivity.

**Table 1**

| Entry | t (h) | Catalyst | Solvent | T (°C) | Yield | e.e. (%) |
|---|---|---|---|---|---|---|
| 1 | 15 | 4 | DCM | 0 | 98 | 83 |
| 2 | 15 | 4 | CHCl₃ | 0 | 98 | 73 |
| 3 | 15 | 4 | DCM | -20 | 98 | 66 |
| 4 | 15 | 4 | CHCl₃ | -20 | 98 | 71 |
| 5 | 15 | 5 | DCM | 0 | 98 | 83 |
| 6 | 15 | 5 | CHCl₃ | 0 | 98 | 73 |
| 7^{a} | 15 | 4 | DCM | 0 | 85 | 67 |

### Example 7. Preparation of N-(1-phenylethyl)benzylamine

*N-*(1-phenylethyl)benzylamine was prepared according to the general procedure from *N*-benzyl imine of acetophenone in quantitative yield and 85% ee in dichloromethane at 0°C using the catalyst of example 2.

### Example 8. Preparation of 2-phenyl piperidine

2-Phenyl piperidine was prepared according to the general procedure from 2-phenyl tetrahydropyridine using the compound of example 2 as catalyst, obtaining the product in 61% yield and 63% ee.

### Example 9. Preparation of N-(4-methoxyphenyl)-phenyl glycine methylester

*N*-(4-methoxyphenyl)-phenyl glycine methylester was prepared according to the general procedure from *N*-(4-methoxyphenyl)-imine of phenylglyoxalate methylester using the compound of example 2 at 0°C in dichloromethane in 98% yield and 41% ee.

### PART C - REDUCTION OF KETIMINES WITH COMPOUNDS (IX)

The reduction is carried out according to general procedure reported in part B, using a catalytic amount of a compound (IX) (0.1-0.01 mol/eq). The following examples refer in particular to the use of *N-*methyl, *N-*picolinoyl-(1*R*, 2*S*)-ephedrine.

### Example 10. Preparation of N-(1-phenylethyl)-butylamine

The reduction of *N-*butylimine of acetophenone was was performed in the presence of trichlorosilane (1.5 - 3 mol/eq) and of a catalytic amount (1%) of *N*-methyl, *N*-picolinoyl-(1*R,* 2*S*)-ephedrine, under different experimental conditions; the best results were obtained in chlorinated solvents (Table 2). *N*-methyl, *N-*picolinoyl-(1*R*, 2*S*)-ephedrine, was able to promote the reduction in dichloromethane at 0°C in quantitative yield and 77% ee. Lowering the reaction temperature to 20°C, enantioselectivity increased to 82% without decrease of the chemical yield. Working in chloroform, a further improvement in enantioselection was observed (97% yield and 90% ee).

**Table 2**

| Entry | t (h) | Solvent | T(°C) | Yield | e.e. (%) |
|---|---|---|---|---|---|
| 1 | 15 | DCM | 0 | 98 | 77 |
| 2 | 15 | CHCl₃ | 0 | 97 | 90 |
| 3 | 15 | DCM | -20 | 98 | 82 |
| 4 | 15 | CHCl₃ | -20 | 98 | 83 |
| 5^{a} | 40 | CHCl₃ | 0 | 77 | 71 |

### Example 11. Preparation of N-(1-phenylethyl)benzylamine

*N*-methyl, *N*-picolinoyl-(1*R*, 2*S*)-ephedrine, reduced *N*-benzyl imine of acetophenone to *N-*(1-phenylethyl)benzylamine in quantitative yield and 75% ee carrying out the reaction according to the general procedure in dichloromethane at 0°C.

### Example 12. Preparation of 2-phenyl piperidine

*N*-methyl, *N*-picolinoyl-(1*R*, 2*S*)-ephedrine reduced 2-phenyl tetrahydropyridine to 2-phenyl piperidine in 98% yield and 71% ee in dichloromethane at 0°C.

## Claims

1. Compounds of general formula (I) 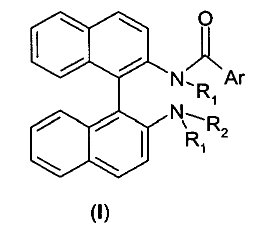 wherein:
R₁ is straight or branched (C₁-C₄)alkyl or benzyl optionally substituted with one or more straight or branched (C₁-C₄)alkyl or (C₁-C₄)alkoxy, or with one or more halogens selected from fluorine, chlorine, bromine and iodine, which can be the same or different from one another;
R₂ is straight or branched (C₁-C₄)alkyl or COR₃, in which R₃ is straight or branched (C₁-C₄)alkyl optionally substituted with one or more halogen atoms selected from fluorine, chlorine, bromine and iodine, phenyl, pyridine, quinoline or isoquinoline optionally optionally substituted with one or more halogens selected from fluorine, chlorine, bromine and iodine, (C₁-C₄)alkyl-or (C₁-C₄)alkoxy;
Ar is pyridine, quinoline or isoquinoline optionally substituted with one or more halogens selected from fluorine, chlorine, bromine and iodine, straight or branched (C₁-C₄)alkyl or straight or branched (C₁-C₄)alkyloxy which may be the same or different from one another.

2. A compound according to claim 1 wherein Ar and R₃ are selected pyridin-2-yl, quinolin-2-yl and isoquinolin-1-yl.

3. A compound selected from: 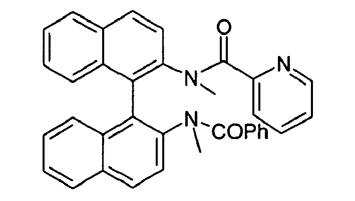 and

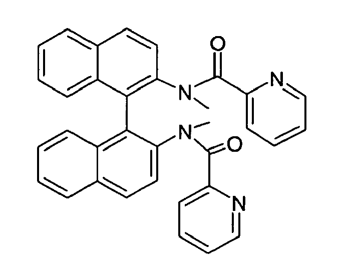

4. A process for the preparation of a compound of formula (I) according to claim 1 wherein R₁ is as defined in claim 1 and R₂ is straight or branched (C₁-C₄)alkyl which comprises the reaction of a chiral diamine of formula (IIa): 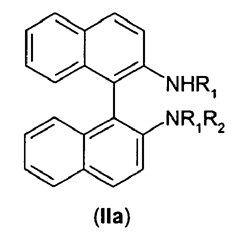 with an equimolar amount of an activated carboxylic acid Ar-COX, wherein Ar is as defined in claim 1 and X is a carboxy-activating group.

5. A process for the preparation of a compound of formula (I) according to claim 1 wherein R₁ is as defined in claim 1 and R₂ is R₃COX, wherein R₃ is as defined in claim 1 and X is a carboxy-activating group, which comprises the reaction of a chiral diamine of formula (IIb) 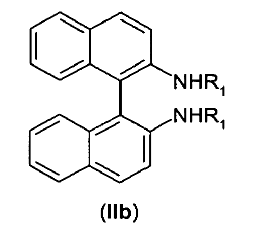 with an equimolar amount of an activated carboxylic acid Ar-COX followed by an equimolar amount of an activated carboxylic acid R₃COX, wherein R₃ is as defined in claim 1 and X is a carboxy-activating group.

6. A process for reducing a chiral ketimine to a corresponding chiral amine which comprises the reduction of a chiral ketimine with trichlorosilane in the presence of a catalytic amount of a compound of formula (I) as defined in any one of claims 1 to 3.

7. The process of claim 6 wherein the chiral ketimine is a ketimine of formula (IV) 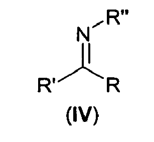 and the amine is an amine of formula (III) 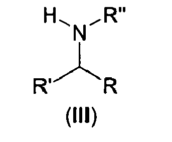 in which
R is straight or branched (C₁-C₄)alkyl;
R' is phenyl or naphthyl optionally substituted with one or more groups selected from fluorine, chlorine, bromine and iodine, (C₁-C₄)straight or branched alkyl or (C₁-C₄)straight or branched alkoxy groups, which may be the same or different from one another; or R' is straight or branched (C₁-C₄)alkyl wherein the carbon chain is optionally interrupted by an oxygen atom;
R" is straight or branched (C₁-C₄)alkyl or benzyl optionally substituted with one or more straight or branched (C₁-C₄)alkyl or (C₁-C₄)alkoxy, or with one or more halogen atoms selected from fluorine, chlorine, bromine and iodine, which can be the same or different from one another.

8. The process of claim 7 wherein the chiral ketimine (IV) is prepared *in situ* by reaction of a ketone RCOR' and an amine R"NH₂ wherein R, R' and R" are as defined in claim 7.

9. The process of claim 6 wherein the ketimine is a cyclic imine of formula (VI) 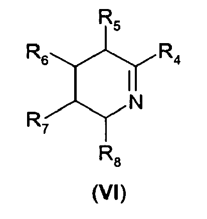 and the amine is a cyclic amine of formula (V) 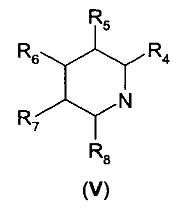 in which R₄-R₈ are independently selected from hydrogen, straight or branched (C₁-C₄)alkyl or phenyl, or any two adjacent R₄-R₈ groups can be reciprocally linked to form a six-membered aromatic ring.

10. The process of claim 9 wherein ketimine (VI) is prepared *in situ* by cyclization of a γ-amino-ketone of formula (VII):

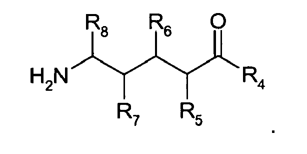

11. The use of the compounds of formula (I) as defined in any one of claims 1 to 3, as catalysts for the reduction of chiral ketimines in the presence of trichlorosilane.

12. A process for reducing a *N*-alkyl- or *N*-benzyl-ketimine to a corresponding chiral *N*-alkyl- or *N*-benzyl amine which comprises the reduction of a ketimine with trichlorosilane in the presence of a catalytic amount of the (1*R*,2*S*)- or (1*S*,2*R*)-isomer of at least one compound of formula (IX): 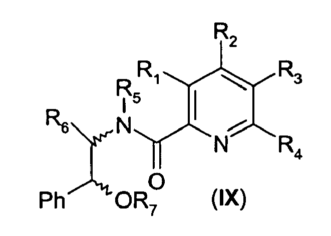 wherein
R₁ is H, halogen or (C₁-C₄)straight or branched alkyl;
R₂ is H or halogen;
R₃ is H or halogen;
R₄ is H or halogen;
R₅ is (C₁-C₄)straight or branched alkyl, or benzyl optionally substituted with one or more halogens or (C₁-C₄)straight or branched alkyl groups, which can be the same or different from one another;
R₆ is (C₁-C₄)straight or branched alkyl, or R₆ can be linked to the adjacent 1-phenyl group to form a ring of formula A: 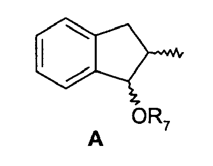
and R₇ is H, (C₁-C₄)straight or branched alkyl, or a CO-aryl group, wherein aryl is selected from phenyl, naphthyl, thiophenyl, furyl, oxazolyl, thiazolyl and pyridyl.

13. The process according to claim 12 wherein in the compound of formula (IX) R₁-R₄ are hydrogen, R₅ and R₆ are methyl and R₇ is hydrogen.

14. The process according to claim 12 or 13 wherein the *N*-alkyl- or *N-*benzyl-ketimines have formulae (IV) and (VI) as defined in claims 7 and 9 respectively.

15. The process according to any one of claims 12-14 wherein the *N*-alkyl-or *N*-benzyl-ketimines are prepared *in situ*.

16. The use of the compounds of formula (IX) as defined in claim 12 as catalysts for the reduction of chiral N-alkyl or N-benzyl ketimines in the presence of trichlorosilane.
